# EUROPEAN PATENT APPLICATION

(11) **EP 1 972 610 A1**
(43) Date of publication of application: **24.09.2008**
(21) Application number: 07713599.4
(22) Date of filing: 12.01.2007
(51) Int. Cl.: C07C 43/13, C07C 41/26

(54) **FLUORINATED ETHER ALCOHOL AND PROCESS FOR PRODUCTION THEREOF**

(30) Priority: 13.01.2006 JP 2006005696
(71) Applicant: Unimatec Co., Ltd., Tokyo 105-8585 (JP)
(72) Inventor: MURATA, Seiichiro, Ibaraki 319-1544 (JP); KOKIN, Keisuke, Ibaraki 319-1544 (JP); SONOI, Takehiro, Ibaraki 319-1544 (JP)
(74) Representative: Albrecht, Thomas
(86) International application number: PCT/JP2007/050292
(87) International publication number: WO 2007/080949

(57) **Abstract**

A novel fluorine-containing ether alcohol having a terminal perfluoromethoxy group represented by the following general formula :

CF₃O[CF(CF₃)CF₂O]ₙCF(CF₃)CH₂OH

(where n is an integer of 0-12) can be produced by reduction reaction of a fluorine-containing ether carboxylic acid alkyl ester represented by the following general formula :

CF₃O[CF(CF₃)CF₂O]ₙCF(CF₃)COOR

(where R is an alkyl group, and n is an integer of 0-12), and the fluorine-containing ether alcohol alone or as a mixture with a fluorine-containing ether alcohol having a terminal perfluoroethoxy group or a terminal perfluoropropoxy group is expected to show a peculiar surfactant characteristic owing to the peculiarity of reaction at the terminal position, and can be used as a dispersing agent or a levelling agent.

## Description

### TECHNICAL FIELD

The present invention relates to a fluorine-containing ether alcohol, and a process for producing the same, and more particularly a fluorine-containing ether alcohol having a perfluoromethoxy group at the molecule terminal, and a process for producing the same.

### BACKGROUND ART

Fluorine-containing ether alcohol is considered to have wide utilization as surfactants, etc. Terminal group of the so far reported fluorine-containing ether alcohols is a perfluoroethoxy group or perfluoropropoxy group. No fluorine-containing ether alcohol having a terminal perfluoromethoxy group has been so far disclosed yet.
Patent Literature 1 : JP-A-58-90524
Patent Literature 2 : JP-A-2003-12606

Fluorine-containing ether alcohol having a terminal perfluoromethoxy group alone or as a mixture with a fluorine-containing ether alcohol having a terminal perfluoroethoxy group or a terminal perfluoropropoxy group is expected to have a peculiar surfactant characteristic owing to peculiarity of reaction at the terminal position.

### DISCLOSURE OF THE INVENTION

### PROBLEM TO BE SOLVED BY THE INVENTION

An object of the present invention is to provide a novel fluorine-containing ether alcohol having a terminal perfluoromethoxy group, and a process for producing the same.

### MEANS FOR SOLVING THE PROBLEM

The present invention provides a fluorine-containing ether alcohol represented by the following general formula :

CF₃O[CF(CF₃)CF₂O]ₙCF(CF₃)CH₂OH

(where n is an integer of 0-12), which can be produced by reduction reaction of a fluorine-containing ether carboxylic acid alkyl ester represented by the following general formula :

CF₃O[CF(CF₃)CF₂O]ₙCF(CF₃)COOR

(where R is an alkyl group and n is an integer of 0-12).

### EFFECT OF THE INVENTION

The present invention provides a novel fluorine-containing ether alcohol having a terminal perfluoromethoxy group, which can be readily produced by allowing carbonyl fluoride to react with hexafluoropropylene oxide, then allowing the resulting carboxyl acid fluoride to react with alcohol, and subjecting the resulting corresponding fluorine-containing ether carboxylic acid ester to reduction reaction. Owing to the peculiarity of reaction at the terminal position, the present fluorine-containing ether alcohol having a terminal perfluoromethoxy group alone or as a mixture with a fluorine-containing ether alcohol having a terminal perfluoroethoxy group or a terminal perfluoropropoxy group is expected to show a peculiar surfactant characteristic and can be used as a dispersing agent or a levelling agent.

### BEST MODES FOR CARRYING OUT THE INVENTION

Fluorine-containing ether carboxylic acid alkyl ester as a raw material for synthesis of the fluorine-containing ether alcohol can be produced by allowing carbonyl fluoride COF₂ with hexafluoropropylene oxide [HFPO] in the presence of a catalyst such as KF, etc., and then allowing the resulting carboxylic acid fluoride to react with alcohol according to the conventional process. These series of reactions include the case of n=0 :

COF₂ + HFPO→CF₃O[CF(CF₃)CF₂O]ₙCF(CF₃)COF + ROH →CF₃O[CF(CF₃)CF₂O]ₙCF(CF₃)COOR

The alcohol for use in the reaction is not particularly limited, but in the view of handling, cost, etc., alcohols having 1-12 carbon atoms, preferably 1-4 carbon atoms, are preferable. Particularly preferable is methanol or ethanol. Hydrogen fluoride is by-produced during the reaction, so an equivalent weight of a metal fluoride or an amine to the raw material acid fluoride may be present in the reaction system as a hydrogen fluoride-trapping agent. Particularly in view of handling, sodium fluoride is preferable for use.

Reduction of the resulting fluorine-containing carboxylic acid alkyl ester can be carried out with an ordinary reducing agent such as aluminum lithium hydride, diisobutyl aluminum lithium hydride, boron sodium hydride, boron cyanosodium hydride, diborane, etc. In view of the safety, reducibility, cost, etc., boron sodium hydride is particularly preferable for use. Boron sodium hydride, which is used generally in a proportion of about 0.5 to about 1% by mole on the basis of the fluorine-containing ether carboxylic acid alkyl ester, can be used in a solid state or in a state of an aqueous basic solution, because there is no difference in the reducibility therebetween.

For reduction reaction, a solvent can be used, if desired. The solvent includes, for example, alcohols such as methanol, ethanol, isopropanol, etc., and ethers such as tetrahydrofuran, dioxane, diethyl ether, etc. Ethanol and isopropanol are particularly preferable for use. A wide range of reaction temperature can be used in view of n in the general formula of the raw material fluorine-containing ether carboxylic acid alkyl ester or the boiling point of alcohol solvent, and a range of about 20° to about 30°C is preferable in view of easy control.

Fluorine-containing ether alcohols as reduction reaction products can have an integer of 0-12 for n, and n=1-5 is preferable from the viewpoint of easy production. For example, the following compounds are included :

CF₃OCF(CF₃)CH₂OH

CF₃O[CF(CF₃)CF₂O]CF(CF₃)CH₂OH

CF₃O[CF(CF₃)CF₂O]₂CF(CF₃)CH₂OH

CF₃O[CF(CF₃)CF₂O]₃CF(CF₃)CH₂OH

### EXAMPLE

The present invention will be described in detail below, referring to Example.

EXAMPLE
187g (0.30mole) of CF₃O[CF(CF₃)CF₂O]₂CF(CF₃)COOCH₃ (98GC%) and 123g of ethanol were charged into a 4-necked flask having a capacity of 300ml and provided with a condenser, a stirrer, and a thermometer, and then thoroughly mixed together by stirring, and 10g (0.26moles) of boron sodium hydride was added thereto portion by portion, while ice cooling the flask, followed by stirring for one hour, while keeping the temperature at not more than 30°C. Dilute hydrochloric acid was then added to the reaction mixture, whereby the resulting lower organic layer was recovered, and washed with water. The organic layer was dried over magnesium sulfate, whereby 169g of colorless, transparent liquid (92GC%; synthesis yield 94%) at the ordinary temperature was obtained. It was confirmed by ¹H-NMR and ¹⁹F-NMR that the liquid was comprised of the following fluorine-containing ether alcohol :

CF₃O[CF(CF₃)CF₂O]₂CF(CF₃)CH₂OH

¹H-NMR((CD₃)₂CO, TMS); δ : 4.28(CH₂), 5.33(OH)
¹⁹F-NMR((CD₃)₂CO, CFCl₃) ; -52.55ppm(CF₃O)
; -79.03ppm(CF₃CFCF₂)
; -80.87ppm(CF₃CFCH₂)
; -133.66ppm(CFCH₂)
; -144.13ppm(CFCF₂)
; -144.96ppm(CFCF₂)

The colorless, transparent liquid was subjected to simple distillation under such conditions as internal pressure : 3-4mmHg (400-533Pa), internal temperature : 52° -53°C, and distillation tower top temperature : 50° -52°C, whereby 153g of the desired product was obtained (99.8GC%, distillation yield 98.2%).

## Claims

1. A fluorine-containing ether alcohol represented by the following general formula :
CF₃O[CF(CF₃)CF₂O]ₙCF(CF₃)CH₂OH
(where n is an integer of 0-12).

2. A fluorine-containing ether alcohol according to Claim 1, wherein the compound represented by the following general formula :
CF₃O[CF(CF₃)CF₂O]ₙCF(CF₃)CH₂OH
is
CF₃OCF(CF₃)CH₂OH,
CF₃O[CF(CF₃)CF₂O]CF(CF₃)CH₂OH,
CF₃O[CF(CF₃)CF₂O]₂CF(CF₃)CH₂OH,
or
CF₃O[CF(CF₃)CF₂O]₃CF(CF₃)CH₂OH.

3. A process for producing a fluorine-containing ether alcohol represented by the following general formula :
CF₃O[CF(CF₃)CF₂O]ₙCF(CF₃)CH₂OH
(where n is an integer of 0-12), **characterized by** subjecting a fluorine-containing ether carboxylic acid alkyl ester represented by the following general formula :
CF₃O[CF(CF₃)CF₂O]ₙCF(CF₃)COOR
(where R is an alkyl group having 1-12 carbon atoms, and n is an integer of 0-12) to reduction reaction.

4. A process for producing a fluorine-containing ether alcohol according to Claim 3, wherein the reduction reaction is carried out in the presence of a boron sodium hydride catalyst.
